# EUROPEAN PATENT SPECIFICATION

(11) **EP 1 409 044 B1**
(45) Date of publication and mention of the grant of the patent: **12.09.2007**
(21) Application number: 02746632.5
(22) Date of filing: 15.07.2002
(51) Int. Cl.: A61M 5/28, A61M 5/315, A61M 37/00

(54) **CARTRIDGE-FREE, MULTI-DOSE INJECTION APPARATUS**
KARTUSCHENLOSE MULTIDOSIS-INJEKTIONSVORRICHTUNG
APPAREIL D'INJECTION MULTI-DOSE SANS CARTOUCHE

(30) Priority: 16.07.2001 US 305733 P
(43) Date of publication of application: 21.04.2004
(73) Proprietor: ELI LILLY AND COMPANY, Indianapolis, Indiana 46285 (US)
(72) Inventor: FISHER, Mark, James, Highland Park, IL 60035 (US); NESBITT, Robert, Fishers, IN 46038 (US)
(74) Representative: Kent, Lindsey Ruth
(86) International application number: PCT/US2002/019814
(87) International publication number: WO 2003/008022

(56) References cited:
- EP-A- 0 897 729
- WO-A-01/10484
- GB-A- 2 249 727
- US-A- 5 211 285
- US-A- 5 300 041

## Description

### BACKGROUND OF THE INVENTION

The present invention pertains to medication dispensing devices, and, in particular, to a portable injection apparatus such as an injection pen.

Patients suffering from a number of different diseases frequently must inject themselves with medication. To allow a person to conveniently and accurately self-administer medicine, a variety of injecting devices broadly known as injector pens or injection pens have been developed. Typically, these pens hold a cartridge having a glass housing and including a piston and containing a multi-dose quantity of liquid medication. A drive member, extending from within a base of the injection pen and operably connected with typically more rearward mechanisms of the pen that control drive member motion, is movable forward to advance the piston in the cartridge in such a manner to dispense the contained medication from an outlet at the opposite cartridge end, typically through a needle that penetrates a stopper at that opposite end. In disposable pens, which are also known as prefilled pens, after a pen has been utilized to exhaust the supply of medication within the cartridge, the entire pen is discarded by a user, who then begins using a new replacement pen.

While useful, cartridge-based injection devices are not without their shortcomings. For example, in prefilled pens, the assembly of a separate glass-housing cartridge into a protective pen housing not only involves a step in the manufacturing process, but also increases the dimensions as well as weight of the device.

In a disclosed injection pen system that does not employ a separate glass cartridge within a protective housing, a plastic cartridge is provided that attaches directly to the end of the housing in which the drive mechanism of the device is housed. While this alternate system may be advantageous in some respects, the number of component parts requiring manufacture and assembly during production still may be greater than desirable.

US 5,211,285 describes a telescoping, pharmaceutical mixing container, which may be used with a metering assembly.

It would be desirable to provide an apparatus that can overcome one or more of the shortcomings of the prior art.

### BRIEF SUMMARY OF THE INVENTION

The present invention encompasses a prefilled injection apparatus for multiple dosings of medication, which apparatus integrates conventional cartridge features into an extension of the protective housing of the apparatus, which housing holds the mechanical drive mechanism used to force the medication from the apparatus, thereby eliminating the need for using a separate cartridge.

In one form thereof, the present invention provides a cartridge-free, multi-dose injection apparatus including an external housing including a tubular body, the external housing body defining a hollow interior having a proximal end and a distal end, a multi-dose quantity of medicine within a distal end portion of the external housing and sealed between a movable piston and a septum, a drive member extending within the hollow interior for advancing the piston, a manual actuator external to the housing in the form of a dosage knob for setting the dose to be injected, and a mechanical drive assembly extending within a proximal end portion of the external housing and operably connected with the dosage knob, the mechanical drive assembly being operable by shifting the dosage knob from a first position to a second position, said mechanical drive assembly operable to cause said drive member to advance in the distal direction to shift the piston distally for dispensing a dose of the medicine through an opening in the septum. The external housing body, along at least a portion of its periphery, has a one-piece plastic construction extending from the proximal end to the distal end. The piston slides within the hollow interior in a fluid tight engagement with an internal surface of the external housing body. At least a majority of a length of the mechanical drive assembly fits within the hollow interior of the external housing body at least when the dosage knob is disposed in the second position.

In still another form thereof, the present invention provides a method of making a cartridge-free, multi-dose injection apparatus, including the steps of: forming from plastic an external housing having a tubular body, the tubular housing body having a distal end and a proximal end, the tubular body having an interior surface defining a hollow interior; installing a first sealing member that sealingly engages the tubular housing body either at the distal end of the tubular housing body or at a point within the hollow interior and along the tubular housing body length between the distal and proximal ends; filling a distal portion of the tubular housing body hollow interior with a multi-dose quantity of medicine; after the filling of the medicine, installing a second sealing member that sealingly engages the tubular housing body at the other of the distal end of the tubular housing body and the point within the hollow interior and along the tubular housing body length between the distal and proximal ends, whereby the multi-dose quantity of medicine is sealed directly within the tubular housing body between the first and second sealing members; and installing an mechanical drive assembly and a drive member in a distal portion of the tubular housing body hollow interior after the filling of the medicine, the mechanical drive assembly operable by an dosage knob for setting the dose to be injected and being external to the hollow interior, said mechanical drive assembly operable to cause said drive member to advance in the distal direction to shift one of the first and second sealing members within the tubular housing body toward the other of the first and second sealing members for dispensing a dose of the medicine.

One advantage of the present invention is that a medication injection apparatus can be provided which may facilitate production by reducing the number of component parts requiring manufacture and assembly.

Another advantage of the present invention is that a medication injection apparatus can be provided which is slimmer and lighter weight in design than some other injection apparatuses.

Still another advantage of the present invention is that a medication injection apparatus can be provided with a simpler design that may reduce overall costs.

Yet another advantage of the present invention is that a medication injection apparatus can be provided in which the drug-containing part is injection molded from plastic with tight tolerances, resulting in the apparatus likely being more predictable in terms of certain operational characteristics, such as force required to eject the medication therefrom.

Another advantage of the present invention is that a medication injection apparatus can be provided which, due to the use of a plastic medicine container and the reduction of parts, may improve dose accuracy.

### BRIEF DESCRIPTION OF THE DRAWINGS

The above-mentioned and other advantages and objects of this invention, and the manner of attaining them, will become more apparent, and the invention itself will be better understood by reference to the following description of embodiments of the invention taking in conjunction with the accompanying drawings, wherein:
Fig. 1 is a front elevational view of a first embodiment of a medication injection apparatus of the present invention;
Fig. 2 is a longitudinal cross-sectional view of the medication injection apparatus of Fig. 1 prior to the mounting of a needle assembly, and wherein an actuator, a drive member, and an injecting assembly that converts actuator input into drive member advancement are diagrammatically shown;
Fig. 3 is a longitudinal cross-sectional view of the medication injection apparatus of Fig. 1 at an early stage of its construction;
Fig. 4 is a view similar to the view of Fig. 3, at a later stage of construction; and
Fig. 5 is a view similar to the view of Fig. 4 at a later stage of construction.

Corresponding reference characters indicate corresponding parts throughout the several views. Although the drawings represent an embodiment of the present invention, the drawings are not necessarily to scale, and certain features may be exaggerated or omitted in some of the drawings in order to better illustrate and explain the present invention.

### DETAILED DESCRIPTION OF THE INVENTION

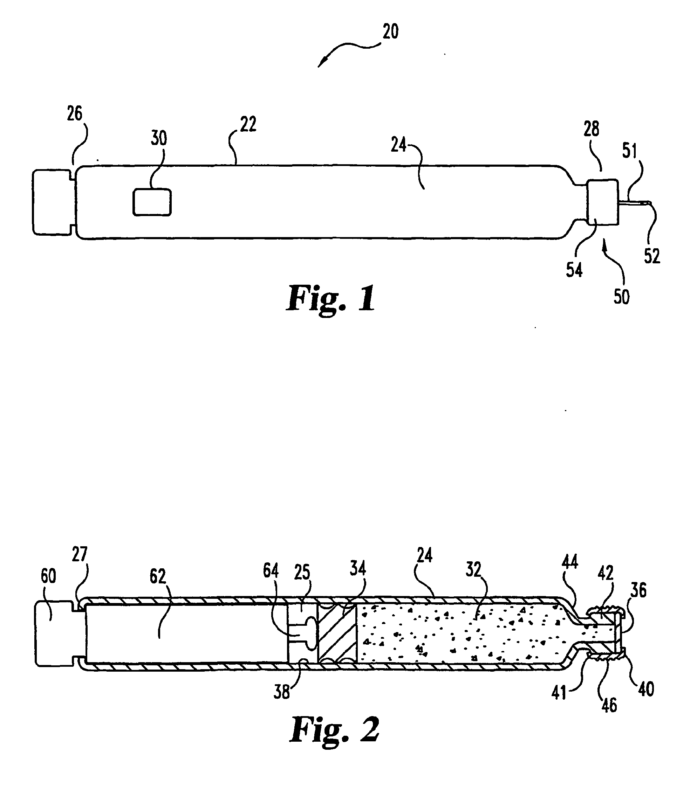

Referring now to Figs. 1 and 2, there is shown a first embodiment of a cartridge-free, multi-dose medication injection apparatus of the present invention. Any directional references in this detailed description with the Figures, such as up or down, are intended for convenience of description, and by itself does not limit the present invention or any of its components to any particular positional or spatial orientation.

The apparatus, generally designated 20, is shown as an injection pen, which pen has an elongated, substantially writing instrument-like form, although other forms are within the scope of the invention. Medication injection pen 20 is a prefilled or disposable pen, in that after the quantity of medicine contained therein is exhausted by multiple operations of the pen, the entire pen is discarded.

Injection pen 20 includes an external, protective housing 22. In the shown embodiment, the housing is provided entirely by a cylindrical tubular body 24 extending from a proximal end 26 to a distal end 28. Body 24 is formed in one-piece, such as by injection molding, out of a polymeric or plastic material that is suitable for use with the medicine it directly contacts and holds as described below. Body 24 may be transparent to allow the contained medicine to be visible to let a user estimate the amount remaining, and the portion surrounding the pen mechanics may have label materials molded therein. Opening 30 in body 24, which may be covered by a magnifying lens, allows for a dose display to be visible to a user. In an alternate embodiment, body 24 may be fashioned in a similar shape from a pair of mating, longitudinally extending pieces that before medicine filling are fixedly secured together, such as by ultrasonic welding or adhesives, but a one-piece body construction is preferred for minimizing parts needed to be manufactured and assembled. Although external housing 22 consists of body 24 in the shown embodiment, one or more additional housing parts, such as an annular cap for the proximal end of the pen body if desired, may also be employed within the scope of the invention.

Sealed within the hollow interior 25 of tubular body 24 at the distal portion of the housing 22 is a multi-dose quantity of a fluid medicine 32 to be delivered by operations of pen 20. Medicine 32 may be any of a variety of drug products, such as diabetes medicines such as insulins, which do not react with the housing. A quantity of 1.5ml or 3.0ml of insulin provides for at least several maximum doses of the typical injection pen, and many multiples thereof for smaller dose amounts. Medicine 32 is sealed directly within housing body 24 between a movable piston 34 and a stopper or septum 36. Piston 34, which may be made of an elastomeric material, axially slides within hollow interior 25 along a middle section of the housing body length. Piston 34 has a sealing periphery in a fluid tight engagement with the hollow interior-defining internal surface or wall 38 of housing body 24 to contain the fluid medication.

Septum 36 is made of an elastomeric material and serves to seals against external housing body 24 to cover the distal, outlet end of hollow interior 25 and thereby contain medication 32. Septum 36 is held in place by an apertured cap 40 fit thereover and secured to housing body 24. In the shown embodiment, cap 40 is a made of metal and is secured via a depending collar 41 crimped over a radially outwardly projecting annular lip 42 integrally formed on a stepped-down diameter neck region 44 of the housing body. In alternate embodiments, the cap may be otherwise formed, such as made of plastic with its depending collar staked or otherwise secured to housing body 24.

The exterior surface of collar 41 is integrally provided with at least one attachment module indicated at 46 adapted to removably mount a disposable needle assembly, generally designated 50, shown in Fig. 1. Module 46 is shown as a continuous encircling thread formed in collar 41, but other suitable connection means may be provided.

Needle assembly 50 is of known design and includes a double-ended needle cannula or injection needle 51 having a distal tip 52 at one end and a not shown proximal point at the other. Injection needle 51 is mounted in a tubular hub 54 that is screwable onto and off of cap threading 46. Although the needle assembly is shown as having a single injection needle, needle assemblies which may be used with pen 20 may be of various types known in the art, including, but not limited to, assemblies with one or more shortened injection needles, including microneedle arrays.

When needle assembly 50 is mounted on threading 46 as shown in Fig. 1, the proximal point of injection needle 51 extends through the aperture of cap 40, and penetrates and thereby provides an opening in cartridge septum 36 to provide a fluid flow outlet by which medicine within hollow interior 25 can be dispensed through needle 51 when piston 34 is moved toward the needle assembly 50 in a conventional manner during injecting use of pen 20.

Positioned proximally of piston 34 are the components that function, in a preferred embodiment, to allow any one of a number of quantities of medicine to be selected and then expelled from the pen by a user. As further diagrammatically represented in Fig. 2, these components include a manually operable actuator in the form of a dosage knob 60 that projects from the proximal end of pen housing 22, a mechanical drive assembly 62 operably connected to knob 60 and which extends within the proximal end portion of the housing body 24, and a drive member 64 extending in an axial or longitudinal direction within hollow interior 25 and abutting piston 34. Depending on the workings of drive assembly 62, drive member 64 may be, for example, a rotating screw or a rotatably fixed, axially shiftable toothed shaft.

Dosage knob 60 is rotatable in a conventional manner to set the dose to be injected, which rotation causes knob 60 to move to an extended position farther from the proximal end of the housing. When knob 60 is then axially plunged toward the pen housing from this extended first position to a second position shown in Figs. 1 and 2, the drive assembly 62 operates to cause drive member 64 to advance in the distal direction to shift piston 34 distally for dispensing the set dose of the medicine through needle 51. In this shown embodiment, and while particular pieces of the drive assembly 62 may axially move during use, the drive assembly overall is axially secured within the volume indicated at 62 in Fig. 2. Such securement may be achieved by attaching, such as via ultrasonic welding, the drive assembly to the interior wall 38 of tubular body 24, or by mechanically capturing the drive assembly, such as by using a cap at the proximal end of the housing or forming body 24 with an inwardly extending flange 27.

Although dosage knob 60 moves out relative to pen housing 22 when rotated to increase the set dose as described above, and then moves in relative to the pen housing when rotated to decrease a dose set too large, such axial motion during dose setting is a function of the mechanical workings of the pen, and the dosage knob 60 need not so move to fall within the scope of the invention. For example, the dosage knob may be axially fixed when rotated to set the dose, and then plunged farther into the pen housing to inject a dose. In addition, while the element actuated to cause the drive assembly to advance drive member 64 and thereby piston 34 distally is described herein as the same element or knob used to set the dose, the actuating element may be different from the dose setting element within the scope of the invention.

The foregoing general description of the mechanism used to advance piston 34 is intended to be illustrative and not limiting in any way. A variety of injection pens, including pull to prepare/push to inject type injection pens, are known in the art which include different mechanisms that allow setting and administering doses, and the mechanisms may be readily adapted for use in a cartridge-free injection pen as described herein. Still further, if pen 20 were intended to always deliver the same or a fixed dose, the mechanism could be configured to appropriately advance piston 34 to achieve such fixed dose upon each activation of the external actuator.

The entire axial length of drive assembly 62 is protectively housed within tubular body 24 at all times of pen use in the shown embodiment. Lesser length portions of a drive assembly, such as substantially all or a majority of such length, also can fit within hollow interior 25 of external housing body 24 in alternate embodiments of the present invention. Moreover, such fitting within hollow interior 25 need not be at all times of use, as it is within the scope of the invention for part of the drive assembly to be caused to temporarily project farther beyond the proximal end of the housing during, for example, dose setting.

The structure of pen 20 will be further understood in view of the following explanation of one manner of its construction. A clean room environment and sterilizing and the like will naturally be employed through the production process as appropriate to provide a suitable final product.

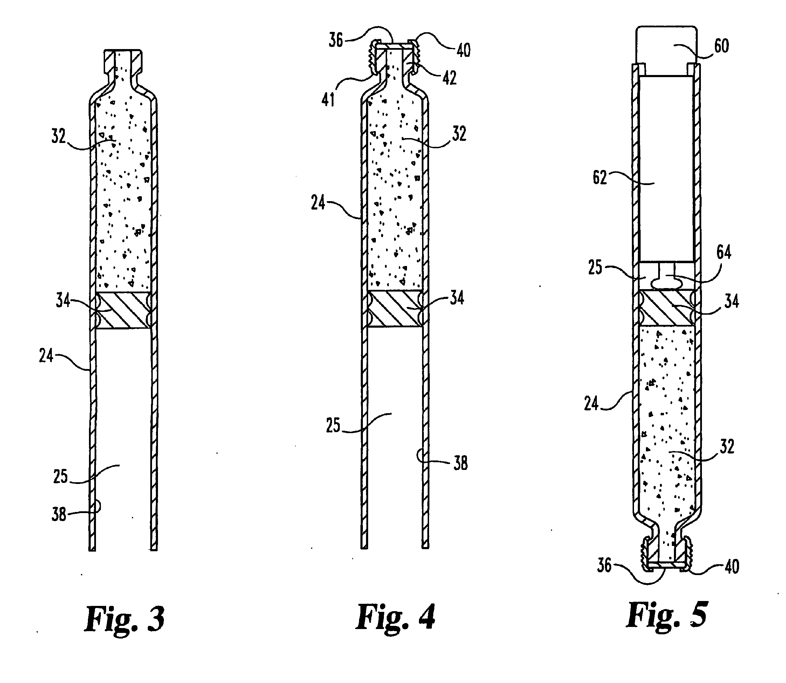

After the external housing 22 is formed from plastic in one-piece by injection molding, piston 34 is installed within hollow interior 25 so as to sealingly engage housing body 24 along a central portion of the body length. With the housing distal end pointing upward, the distal portion of the tubular housing body hollow interior 25 is then filled with a multi-dose quantity of medicine 32. At this stage of the manufacture, the in-progress pen is arranged as shown in Fig. 3.

Next, septum 36 is secured in sealing engagement with the tubular housing body 24 by the crimping of cap 40 onto body lip 42, such that the multi-dose quantity of medicine 32 is now sealed directly within the tubular housing body 24 between piston 34 and septum 36. At this stage of the manufacture, the in-progress pen is arranged as shown in Fig. 4. It will be appreciated that an alternate manufacturing sequence involves sequentially securing the septum, filling the medicine, and then inserting the piston.

The medicine-filled pen housing is then inverted, and the preassembled mechanics of the pen are inserted into the proximal portion of the tubular housing body hollow interior 25, leaving the actuator 60 projecting from the hollow interior. The in-progress pen is now arranged as shown in Fig. 5. After securing the drive assembly 62 to the housing body 24, such as via ultrasonic welding and/or inturned flange 27, the manufacturing is complete, and pen 20 is arranged as shown in Fig. 2.

While this invention has been shown and described as having preferred designs, the present invention may be modified within the scope of the claims. This application is therefore intended to cover any variations, uses or adaptations of the invention using its general principles. Further, this application is intended to cover such departures from the present disclosure as come within known or customary practice in the art to which this invention pertains.

## Claims

1. A cartridge-free, multi-dose injection apparatus (20) comprising:
an external housing (22) including a tubular body (24), said external housing body defining a hollow interior having a proximal end (26) and a distal end (28);
a multi-dose quantity of medicine (32) within a distal end portion of said external housing and sealed between a movable piston (34) and a septum (36);
a drive member (64) extending within said hollow interior for abutting said piston;
a manual actuator (60) external to said housing in the form of a dosage knob for setting the dose to be injected,
a mechanical drive assembly (62) extending within a proximal end portion of said external housing and operably connected with said dosage knob, said mechanical drive assembly operable by shifting said dosage knob from a first position to a second position, said mechanical drive assembly operable to cause said drive member to advance in the distal direction to shift said piston distally for dispensing a dose of the medicine through an opening in said septum;
wherein said external housing body, along at least a portion of its periphery, has a one-piece plastic construction extending from said proximal end to said distal end;
wherein said piston slides within said hollow interior in a fluid tight engagement with an internal surface (38) of said external housing body; and
wherein at least a majority of a length of said mechanical drive assembly fits within said hollow interior of said external housing body at least when said dosage knob is disposed in said second position.

2. The cartridge-free, multi-dose injection apparatus of claim 1 wherein at least substantially all of the length of said mechanical drive assembly is disposed within said hollow interior of said external housing body.

3. The cartridge-free, multi-dose injection apparatus of claim 1 wherein all of the length of said mechanical drive assembly is disposed within said hollow interior of said external housing body.

4. The cartiridge-free, multi-dose injection apparatus of any one of claims 1 to 3 wherein the opening in said septum is provided by a penetrating needle of a disposable needle assembly (50) removably mounted at a distal end of said external housing.

5. The cartridge-free, multi-dose injection apparatus of claim 4 wherein said septum is sealingly mounted to said external housing body to cover said distal end of said hollow interior of said external housing body.

6. The cartridge-free, multi-dose injection apparatus of claim 5 wherein said septum is sealingly mounting to said external housing body by an apertured cap (40) fit over said septum and secured onto said housing body, said cap further comprising at least one attachment module (46) provided on an exterior surface, said at least one attachment module adapted to removably mount the disposable needle assembly.

7. The cartridge-free, multi-dose injection apparatus of claim 6 wherein said cap is crimped onto said housing body.

8. The cartridge-free, multi-dose injection apparatus of any of claims 1 to 7 wherein the entire external housing body is molded as a single piece.

9. A method of making a cartridge-free, multi-dose injection apparatus (20), comprising the steps of:
forming from plastic an external housing (22) having a tubular body (24), said tubular housing body having a distal end (28) and a proximal end (26), said tubular body having an interior surface defining a hollow interior,
installing a first sealing member (34, 36) that sealingly engages said tubular housing body either at the distal end of said tubular housing body or at a point within the hollow interior and along the tubular housing body length between the distal and proximal ends;
filling a distal portion of the tubular housing body hollow interior with a multi-dose quantity of medicine (32);
after the filling of the medicine, installing a second sealing member (34, 36) that sealingly engages said tubular housing body at the other of said distal end of said tubular housing body and the point within the hollow interior and along the tubular housing body length between the distal and proximal ends, whereby said multi-dose quantity of medicine is sealed directly within said tubular housing body between said first and second sealing members; and
installing a mechanical drive assembly (62) and a drive member (64) in a distal portion of the tubular housing body hollow interior after the filling of the medicine, said mechanical drive assembly operable by a dosage knob (60) for setting the dose to be injected and being external to said hollow interior, said mechanical drive assembly operable to cause said drive member to advance in a distal direction to shift one of said first and second sealing members within said tubular housing body toward the other of said first and second sealing members for dispensing a dose of the medicine.

10. The method of claim 9 wherein said first sealing member comprises a piston (34) that sealingly engages said tubular housing body at the point within the hollow interior and along the tubular housing body length between the distal and proximal ends, said piston shiftable distally by advancement of said drive member.

11. The method of claim 10 wherein said step of installing said second sealing member comprises crimping an apertured cap onto said distal end of said tubular housing body over a septum (36) covering an end of said hollow interior.

12. The method of claim 11 further comprising the step of mounting a needle assembly to at least one attachment module (46) provided on the exterior of said apertured cap.

13. The method of any of claims 9 to 12 wherein said tubular housing body is molded from plastic as a single piece.

## Patentansprüche

1. Kartuschenloses Multidosis-Injektionsgerät (20), enthaltend:
ein Außengehäuse (22) mit einem rohrförmigen Körper (24), der einen hohlen Innenraum bestimmt, der ein hinteres Ende (26) und ein vorderes Ende (28) hat;
eine Multidosis-Menge an Medikament (32) in einem vorderen Endabschnitt des Außengehäuses, die zwischen einem beweglichen Kolben (34) und einem Septum (36) eingeschlossen ist;
ein Antriebselement (64), das sich in dem hohlen Innenraum erstreckt, um an dem Kolben anzustoßen;
ein Handbetätigungsglied (60) außerhalb des Gehäuses in Form eines Dosierknopfes zum Einstellen der zu injizierenden Dosis;
eine mechanische Antriebsanordnung (62), die sich in einem hinteren Endabschnitt des Außengehäuses erstreckt und mit dem Dosierknopf wirkungsverbunden ist und durch Verschieben des Dosierknopfs aus einer ersten Position in eine zweite Position betätigbar ist, wobei die mechanische Antriebsanordnung dazu betätigbar ist, ein Vorschieben des Antriebselements nach vorn zu verursachen, um den Kolben nach vorn zu verschieben, um eine Dosis des Medikaments durch eine Öffnung in dem Septum abzugeben;
wobei der Außengehäusekörper längs wenigstens eines Teils seines Umfangs einen einteiligen Kunststoffaufbau hat, der sich von dem hinteren zu dem vorderen Ende erstreckt;
wobei der Kolben in dem hohlen Innenraum in fluiddichter Anlage an einer Innenfläche (38) des Außengehäusekörpers gleitet; und
wobei wenigstens ein größerer Teil der Länge der mechanischen Antriebsanordnung in den hohlen Innenraum des Außengehäusekörpers wenigstens dann passt, wenn der Dosierknopf in der zweiten Position angeordnet ist.

2. Kartuschenloses Multidosis-Injektionsgerät nach Anspruch 1, bei dem wenigstens im Wesentlichen die gesamte Länge der mechanischen Antriebsanordnung innerhalb des hohlen Innenraums des Außengehäusekörpers angeordnet ist.

3. Kartuschenloses Mehrdosis-Injektionsgerät nach Anspruch 1, bei dem die gesamte Länge der mechanischen Antriebsanordnung innerhalb des hohlen Innenraums des Außengehäusekörpers angeordnet ist.

4. Kartuschenloses Multidosis-Injektionsgerät nach einem der Ansprüche 1 bis 3, bei dem die Öffnung in dem Septum durch eine durchstoßende Nadel einer wegwerfbaren Nadelanordnung (50) geschaffen ist, die am vorderen Ende des Außengehäuses lösbar angebracht ist.

5. Kartuschenloses Multidosis-Injektionsgerät nach Anspruch 4, bei dem das Septum abgedichtet an dem Außengehäusekörper angebracht ist, um das vordere Ende des hohlen Innenraums des Außengehäusekörpers abzudecken.

6. Kartuschenloses Multidosis-Injektionsgerät nach Anspruch 5, bei dem das Septum an dem Außengehäusekörper durch eine mit einer Öffnung versehene Kappe (40) abgedichtet angebracht ist, die über dem Septum sitzt und an dem Außengehäusekörper befestigt ist und wenigstens ein Befestigungsmodul (46) aufweist, das an einer Außenfläche vorgesehen ist, wobei dieses wenigstens eine Befestigungsmodul dazu eingerichtet ist, die wegwerfbare Nadelanordnung lösbar zu montieren.

7. Kartuschenloses Multidosis-Injektionsgerät nach Anspruch 6, bei dem die Kappe auf den Gehäusekörper aufgefalzt ist.

8. Kartuschenloses Multidosis-Injektionsgerät nach einem der Ansprüche 1 bis 7, bei dem der gesamte Außengehäusekörper als ein einziges Teil gespritzt ist.

9. Verfahren zum Herstellen eines kartuschenlosen Multidosis-Injektionsgerätes (20), umfassend die Schritte:
Erstellen eines Außengehäuses (22) aus Kunststoff, das einen rohrförmigen Körper (24) hat, der ein vorderes Ende (28) und ein hinteres Ende (26) aufweist und eine Innenfläche hat, die einen hohlen Innenraum bestimmt;
Installieren eines ersten Dichtungselements (34,36), das an dem rohrförmigen Gehäusekörper entweder am vorderen Ende desselben oder an einem Punkt innerhalb des hohlen Innenraums und längs der Erstreckung desselben zwischen den vorderen und hinteren Enden dichtend anliegt;
Füllen eines vorderen Abschnitts des hohlen Innenraums des rohrförmigen Gehäusekörpers mit einer Multidosis-Menge an Medikament (32);
nach dem Füllen mit dem Medikament Installieren eines zweiten Dichtungselements (34,36), das an dem rohrförmigen Gehäusekörper an dem anderen Ende desselben und dem Punkt innerhalb des hohlen Innenraums und längs der Längserstreckung des rohrförmigen Gehäusekörpers zwischen den vorderen und hinteren Enden anliegt, wodurch die Multidosis-Menge an Medikament direkt innerhalb des rohrförmigen Gehäusekörpers zwischen den ersten und zweiten Dichtungselementen eingeschlossen wird; und
Installieren einer mechanischen Antriebsanordnung (62) und eines Antriebselements (64) in einem vorderen Abschnitt des hohlen Innenraums des rohrförmigen Gehäusekörpers nach dem Einfüllen des Medikaments, wobei die mechanische Antriebsanordnung durch einen Dosierknopf (60) zum Einstellen der zu injizierenden Dosis betätigbar ist, der außerhalb des hohlen Innenraums liegt, wobei die mechanische Antriebsanordnung betätigbar ist, um das Antriebselement zu veranlassen, sich nach vorn zu bewegen, um eines der ersten und zweiten Dichtungselemente innerhalb des rohrförmigen Gehäusekörpers gegen das andere der ersten und zweiten Dichtungselemente zu verschieben, um eine Dosis des Medikaments abzugeben.

10. Verfahren nach Anspruch 9, bei dem das erste Dichtungselement einen Kolben (34) umfasst, der dichtend an dem rohrförmigen Gehäusekörper an dem Punkt innerhalb des hohlen Innenraums und längs der Längserstreckung des rohrförmigen Gehäusekörpers zwischen den vorderen und hinteren Enden dichtend anliegt und durch Vorschieben des Antriebselements nach vorn verschiebbar ist.

11. Verfahren nach Anspruch 10, bei dem der Schritt des Installierens des zweiten Dichtungselements das Auffalzen einer mit einer Öffnung versehenen Kappe auf das vordere Ende des rohrförmigen Gehäusekörpers über einem Septum (36) umfasst, das ein Ende des hohlen Innenraums abdeckt.

12. Verfahren nach Anspruch 11, weiterhin umfassend den Schritt des Montierens einer Nadelanordnung an wenigstens einem Befestigungsmodul (46), das an der Außenseite der mit der Öffnung versehenen Kappe vorgesehen ist.

13. Verfahren nach einem der Ansprüche 9 bis 12, bei dem der rohrförmige Gehäusekörper aus Kunststoff als ein einziges Teil gespritzt wird.

## Revendications

1. Appareil d'injection multi-dose sans cartouche (20) comprenant :
un logement externe (22) incluant un corps tubulaire (24), ledit corps de logement externe définissant un intérieur creux ayant une extrémité proximale (26) et une extrémité distale (28) ;
une quantité multi-dose de médicament (32) au sein d'une partie d'extrémité distale dudit logement externe et enfermée entre un piston mobile (34) et un septum (36) ;
un élément d'entraînement (64) s'étendant au sein dudit intérieur creux pour venir buter contre ledit piston ;
un actionneur manuel (60) externe audit logement sous la forme d'un bouton de dosage pour fixer la dose devant être injectée ;
un ensemble d'entraînement mécanique (62) s'étendant au sein d'une partie d'extrémité proximale dudit logement externe et opérationnellement connecté avec ledit bouton de dosage, ledit ensemble d'entraînement mécanique étant utilisable en déplaçant ledit bouton de dosage d'une première position à une deuxième position, ledit ensemble d'entraînement mécanique étant utilisable pour faire avancer ledit élément d'entraînement dans la direction distale pour déplacer ledit piston distalement pour délivrer une dose du médicament par une ouverture dans ledit septum ;
dans lequel ledit corps de logement externe, le long d'au moins une partie de sa périphérie, a une construction en plastique monobloc s'étendant de ladite extrémité proximale à ladite extrémité distale ;
dans lequel ledit piston coulisse au sein dudit intérieur creux en un engagement étanche aux fluides avec une surface interne (38) dudit corps de logement externe ; et
dans lequel au moins une majorité d'une longueur dudit ensemble d'entraînement mécanique s'ajuste au sein dudit intérieur creux dudit corps de logement externe au moins lorsque ledit bouton de dosage est disposé dans ladite deuxième position.

2. Appareil d'injection multi-dose sans cartouche selon la revendication 1 dans lequel au moins sensiblement la totalité de la longueur dudit ensemble d'entraînement mécanique est disposée au sein dudit intérieur creux dudit corps de logement externe.

3. Appareil d'injection multi-dose sans cartouche selon la revendication 1 dans lequel la totalité de la longueur dudit ensemble d'entraînement mécanique est disposée au sein dudit intérieur creux dudit corps de logement externe.

4. Appareil d'injection multi-dose sans cartouche selon l'une quelconque des revendications 1 à 3 dans lequel l'ouverture dans ledit septum est pratiquée par une aiguille pénétrante d'un ensemble d'aiguille jetable (50) monté de façon amovible à une extrémité distale dudit logement externe.

5. Appareil d'injection multi-dose sans cartouche selon la revendication 4 dans lequel ledit septum est monté de façon étanche sur ledit corps de logement externe pour recouvrir ladite extrémité distale dudit intérieur creux dudit corps de logement externe.

6. Appareil d'injection multi-dose sans cartouche selon la revendication 5 dans lequel ledit septum est monté de façon étanche sur ledit corps de logement externe par un bouchon ouvert (40) ajusté par-dessus ledit septum et fixé sur ledit corps de logement, ledit bouchon comprenant en outre au moins un module d'attachement (46) prévu sur une surface extérieure, ledit au moins un module d'attachement étant adapté pour monter de façon amovible l'ensemble d'aiguille jetable.

7. Appareil d'injection multi-dose sans cartouche selon la revendication 6 dans lequel ledit bouchon est serti sur ledit corps de logement.

8. Appareil d'injection multi-dose sans cartouche selon l'une quelconque des revendications 1 à 7 dans lequel la totalité du corps de logement externe est moulée en une pièce.

9. Procédé de fabrication d'un appareil d'injection multi-dose sans cartouche (20), comprenant les étapes de :
former à partir de plastique un logement externe (22) ayant un corps tubulaire (24), ledit corps de logement tubulaire ayant une extrémité distale (28) et une extrémité proximale (26), ledit corps tubulaire ayant une surface intérieure définissant un intérieur creux ;
installer un premier élément d'étanchéité (34, 36) qui s'engage de façon étanche avec le corps de logement tubulaire soit à l'extrémité distale dudit corps de logement tubulaire, soit en un point au sein de l'intérieur creux et le long de la longueur du corps de logement tubulaire entre les extrémités distale et proximale ;
remplir une partie distale de l'intérieur creux de corps de logement tubulaire avec une quantité multi-dose de médicament (32) ;
après le remplissage du médicament, installer un deuxième élément d'étanchéité (34, 36) qui s'engage de façon étanche avec ledit corps de logement tubulaire à l'autre de ladite extrémité distale dudit corps de logement tubulaire et du point au sein de l'intérieur creux et le long de la longueur du corps de logement tubulaire entre les extrémités distale et proximale, d'où il résulte que ladite quantité multi-dose de médicament est enfermée directement au sein dudit corps de logement tubulaire entre lesdits premier et deuxième éléments d'étanchéité ; et
installer un ensemble d'entraînement mécanique (62) et un élément d'entraînement (64) dans une partie distale de l'intérieur creux de corps de logement tubulaire après le remplissage du médicament, ledit ensemble d'entraînement mécanique étant utilisable par un bouton de dosage (60) pour fixer la dose devant être injectée et étant externe audit intérieur creux, ledit ensemble d'entraînement étant mécanique utilisable pour faire avancer ledit élément d'entraînement dans une direction distale pour déplacer l'un dudit premier et dudit deuxième éléments d'étanchéité au sein dudit corps de logement tubulaire vers l'autre dudit premier et dudit deuxième éléments d'étanchéité pour délivrer une dose du médicament.

10. Procédé selon la revendication 9 dans lequel le premier élément d'étanchéité comprend un piston (34) qui s'engage de façon étanche avec ledit corps de logement tubulaire en un point au sein de l'intérieur creux et le long de la longueur du corps de logement tubulaire entre les extrémités distale et proximale, ledit piston étant déplaçable distalement par avancement dudit élément d'entraînement.

11. Procédé selon la revendication 10 dans lequel ladite étape d'installation dudit deuxième élément d'étanchéité comprend de sertir un bouchon ouvert sur ladite extrémité distale dudit corps de logement tubulaire par-dessus un septum (36) recouvrant une extrémité dudit intérieur creux.

12. Procédé selon la revendication 11 comprenant en outre l'étape de monter un ensemble d'aiguille sur au moins un module d'attachement (46) prévu sur l'extérieur dudit bouchon ouvert.

13. Procédé selon l'une quelconque des revendications 9 à 12 dans lequel ledit corps de logement tubulaire est moulé en une pièce à partir de plastique.
